# EUROPEAN PATENT SPECIFICATION

(11) **EP 4 643 340 B1**
(45) Date of publication and mention of the grant of the patent: **15.07.2026**
(21) Application number: 23848248.3
(22) Date of filing: 29.12.2023
(51) Int. Cl.: G16B 20/50, G16B 20/20, G16B 30/10, G16B 30/20, G16B 40/30

(54) **METHOD FOR ERROR CORRECTION IN NUCLEIC ACID SEQUENCING**
VERFAHREN ZUR FEHLERKORREKTUR BEI DER NUKLEINSÄURESEQUENZIERUNG
PROCÉDÉ DE CORRECTION D'ERREUR DANS LE SÉQUENÇAGE D'ACIDES NUCLÉIQUES

(30) Priority: 29.12.2022 IT 202200027138
(43) Date of publication of application: 05.11.2025
(73) Proprietor: Centro Di Riferimento Oncologico, 33081 Aviano (IT)
(72) Inventor: VIT, Filippo, 33100 Udine (IT); BOMBEN, Riccardo, 33080 Porcia (PN) (IT); GATTEI, Valter, 33084 Cordenons (PN) (IT)
(74) Representative: De Tullio, Michele Elio
(86) International application number: PCT/IB2023/063383
(87) International publication number: WO 2024/142013

(56) References cited:
- US-A1- 2016 034 638
- US-A1- 2016 289 753
- US-A1- 2019 360 045
- GUR YAARI ET AL: "Practical guidelines for B-cell receptor repertoire sequencing analysis", GENOME MEDICINE, vol. 7, no. 1, 20 November 2015 (2015-11-20), XP055453727, DOI: 10.1186/s13073-015-0243-2
- NEHA CHAUDHARY ET AL: "Analyzing Immunoglobulin Repertoires", FRONTIERS IN IMMUNOLOGY, vol. 9, 14 March 2018 (2018-03-14), pages 1 - 18, XP055662897, Retrieved from the Internet <URL:https://www.frontiersin.org/articles/10.3389/fimmu.2018.00462/full> DOI: 10.3389/fimmu.2018.00462

## Description

### FIELD OF THE INVENTION

The present invention relates to a method for correcting sequencing errors in a nucleic acid amplification process. In particular, the present invention relates to a method for correcting systematic errors during the sequencing process of nucleic acids from patients affected by CLL, for statistical and prognostic purposes.

### PRESENT STATUS OF THE ART

As known to those skilled in this art, Next Generation Sequencing (NGS) is capable of accurately identifying and quantifying the sites of variation of a nucleic acid sequence in the genome of an organism, including a human, through highly parallelized platforms, as described, for instance, in WO2021053208 and US2021010060. In particular, it is highly desirable for a human to quantify the change of a group of patients genomes over time, so as to provide a detailed chart of the evolution of a genetic disease. This knowledge has a number of applications, including the development of treatment strategies that are both reactive (in that this information can be used for selecting the best treatment regimen of drugs for a single patient) and predictive (in that the probability of appearance of resistant variants can be calculated and incorporated in vaccine and/or pharmacological treatment design strategies).

This particularly stands true for evaluating a rearrangement, or intraclonal diversification (ID) internally to a group of patients affected by Chronic Lymphocytic Leukaemia (CLL), as reported, for example, in WO2018209058. As known to those skilled in the art, B lymphocytes belong to a group of lymphoid cells different from other somatic cells in the human body. During their development, the natural recombination of the V_{H}, J_{H}, D_{H} genes causes somatic variations with respect to the germinal line ("germline") in the variable region of the heavy chain of immunoglobulins (ImmunoGlobulin Heavy chain Variable region or IGHV), thus generating V_{H} - J_{H} - D_{H} allelic combinations that are specific to each individual cell both in sequence and in length. Lymphoma and leukaemias, such as, for example, the chronic lymphocytic leukaemia, originate from malignant mutations of said cellular individuals.

US 2016/289753A1 discloses methods for evaluating sequence variation by correcting for error reads. GUR YAARI ET AL: "Practical guidelines for B-cell receptor repertoire sequencing analysis",GENOME MEDICINE, vol. 7, no. 1, 20 November 2015, relates to sequencing analyses for B-cell receptor repertoire including unique molecualr identifiers and sequence error correction.

However, a number of problems must be dealt with before the NGS technology can be normally used for characterizing so much complex variation models.

The greatest problem concerns detection of the errors introduced during the sequencing and discrimination of such errors from real genetic variations, in that the degree of genetic variation might be in the same order of magnitude as the error rate of the sequencing process. All sequencing methods intrinsically produce errors which are i) *random* if are generated, for example, through incorporation of a wrong nucleotide base by way of the Taq polymerase (wherein the sequence processing software "calls" a base not correctly), or through amplification, wherein the Taq polymerase incorporates a non-complementary nucleotide base while producing the sequencing library or during the sequencing reaction itself, and ii) *systematic,* which are exclusive characteristics of the equipment used and partly depend on the chemical reagents used in the sequencing process and on the protocol adopted for preparing sequence libraries. Above all, the systematic errors are the most problematic ones, because they limit the capability of identifying a true sequence variation in the samples and lead to an overestimate of ID, thus generating false mutations. For this reason, many of the next-generation sequencing platforms presently used, such as, for example, Illumina^{®} Miseq, are reported to have a low specificity or a high false positive rate.

Recently, a use of Unique Molecular Identifiers (UMI) has been adopted in the sequencing platforms available on the market, which allow to correct random errors, thus resulting in a better representation of the subclonal heterogeneity of specific clonotypes, as reported, for example, in WO2018136248 and EP3077539. Therefore, said UMI platforms increase sequencing accuracy, while simultaneously increasing the number of the samples to process for the genome analysis of a population of individuals, as requested, for example, for ID analysis internally to a group of patients affected by CLL for evaluating subclonal heterogeneity. However, these are technologies that are laborious, very expensive, and not easily accessible to most biological laboratories from an economical point of view, and also request informatic media featuring high computation powers. Also, the UMI sequencing methods are not capable of removing systematic errors.

Because of these drawbacks, a need is still felt for providing efficient and reliable methods for removing the systematic errors generated during sequencing operations on genetic materials derived from patients affected by CLL.

The main object of the present invention is to overcome the technical drawbacks described here above.

### SUMMARY OF THE INVENTION

This object is achieved by using the present invention, which, in its main aspect, relates to a method for correcting systematic errors generated with any of the wet methods used for preparing immune repertoire libraries of immunoglobulins, starting from an extraction of genetic material, organized according to a workflow comprising the following steps:
a) generating a plurality of samples processed in parallel by way of a polymerase chain reaction (PCR), said libraries using at least one wet method.
b) purifying and sequencing the products of the polymerase chain reaction for generating sequences of nucleotides;
c) encoding the results of said step b) into two compressed text format files, each corresponding to the two sequencing directions, the forward one (5' - 3') and the reverse one (3' - 5') of every individual amplicon;
d) collecting said text format into consensus sequences;
e) assigning a pathological clone to the sequences thus obtained on the basis of a comparison with reference data banks;
f) correcting said systematic sequencing errors of the libraries, said step f) comprising the following sub-steps:
   g) aligning said text files with the sequencing of reference data banks, for a significant search for homology, account being taken of interruptions/deletions;
   h) generating counting matrices with the frequencies of the individual nucleotides observed depending on their position, in order to identify the mutations present in the immunoglobulins and their frequencies;
   i) eliminating those text files whose frequencies, as set in sub-step h), are lower than a predetermined threshold value, in order to obtain residual sequences;
   j) calculating the "Phred" quality score dependent on every position of said frequencies;
   k) correcting the positions of the nucleotides specific to immunoglobulins having said score lower than a predetermined threshold value, in order to obtain said corrected text files;
   l) differentiating said files according to the plurality of samples which they come from, processed in parallel as per step a);
   m) calculating the inverse Simpson index of said residual sequences.

Advantageously can said method i) provide detailed information on a group of patients affected by CLL having their variable region of the immunoglobulin heavy chain mutated, and ii) provide data on prognosis and modification of the therapeutical plan of said group of patients, thanks to the correction of the systematic sequencing errors.

### BRIEF DESCRIPTION OF THE FIGURES

Characteristics and advantages of the present invention will be more apparent from the following description of preferred embodiments thereof, which is provided for exemplary non-limitative purposes only with reference to the attached Figures, wherein:
Figure 1A schematically shows the amplified genome regions for the method according to the present invention. The arrows indicate the transcription directions of the specific primers used.
Figure 1B schematically shows the lengths of the amplicons generated by the Lymphotrack^{®} methodology and processed according to the method of the present invention. The lengths of the sequences are expressed in terms of nitrogenous base pairs (bp). The arrows indicate the two reading directions, reading 1 and 2 respectively.
Figure 2A schematically shows the bioinformatic workflow adopted by the method according to the present invention for analysing sequencing libraries.
Figure 2B schematically shows the bioinformatic workflow of the present invention for correcting systematic sequencing errors.
Figure 3 schematically shows a flowchart of the principle of the present invention for removing systematic errors.
Figure 4 schematically shows the PCR amplification protocols for generating UMI-labelled sequencing libraries.
Figure 5A schematically shows the bioinformatic workflow adopted for analysing UMI-labelled sequencing libraries.
Figure 5B schematically shows a flow chart of the principle of the present invention for removing systematic errors from UMI-labelled sequencing libraries.
Figure 6 shows the reliability of the subclonal heterogeneity of the clonotypes of the CLL pathological clone, expressed both as a percentage of the variation of the major subclone and as a percentage of the variation of the minor subclones, as determined by using the method of the present invention without correction (A, C) and with correction (B, D) of the systematic errors (n = number of processed samples).
Figure 7 shows the reliability of the subclonal heterogeneity of the clonotypes of the CLL pathological clone, expressed both as a percentage of the frequency of the major subclone and as a percentage of the variation of the minor subclones, as determined by using the sequencing method with generation of UMI-labelled libraries without correction (A, C) and with correction (B, D) of the systematic errors by using the method of the present invention (n = number of processed samples).

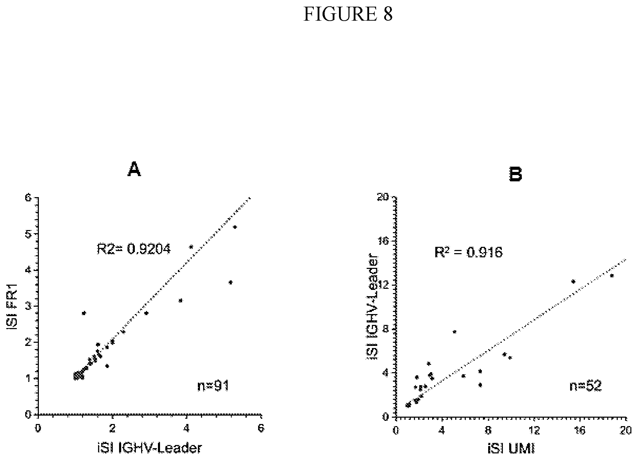

Figure 8 shows a comparison between the inverse Simpson indices obtained with the method of the present invention via (A) the IGHV-LEADER and IGHV-FR1 amplification protocols, and (B) amplification protocols using UMI labels.

### DETAILED DESCRIPTION OF THE INVENTION

Wherever not otherwise specified, all terms used in this description shall be construed according to their ordinary meaning as known to those skilled in the art that the present invention belong to.

The terms and symbols of the chemistry of nucleic acids, biochemistry, genetics and molecular biology as used here, such as, for example, "nucleic acid", "sequence", "nucleotides", "sequencing", "nucleotide base", "5' end", "3' end", "nucleotide sequence", "bp", "homology", "alignment", "gene", "intraclonal diversification", "pathological clone", "subclonal heterogeneity", follow those found in the standard works and text found in the art that the present invention belong to, such as, for example, "Lehninger Principles of Biochemistry", David L. Nelson, Michael M. Cox, fourth edition.

Unless otherwise specified, the mathematical, informatic, and bioinformatic terms provided in the present description, such as, for example, "Phred quality score", and "inverse Simpson index", shall be construed according to their ordinary meanings as known to those skilled in the art that the present invention belongs to. An exhaustive source is "Statistical Methods in Bioinformatics: An Introduction" by Warren J. Evens, Gregory R. Gran, second edition.

Some terms are defined here below for the sake of clarity and ease of reference.

In the present invention, by "amplicon" we mean the product of a polynucleotide amplification reaction, i.e. a population of polynucleotides, usually a double strand one, which are replicated by one or several start sequences. The one or more start sequences can be either one or several couples of the same sequence, or they can be a mix of different sequences. Amplicons can be produced from a variety of amplification reactions whose replicated products are copies of one or several target nucleic acids. Said reactions comprise, for exemplary but non-limitative purposes, polymerase chain reactions (PCR).

By "primer", we mean short sequences of a nucleic acid, usually approx. 20-nucleotide-base long, which indicate which region of the nucleic acid shall be amplified. These are available on the market and are specifically designed for amplifying one or several sequences of target nucleic acids.

By "adapter", we mean a sequence that is added to a nucleic acid (for example, through a chemical reaction). An adapter can be 5 to 100 nucleotide-base long and can provide, for example, an amplification primer binding site, a sequencing primer binding site, and/or a molecular identifier like a sample identifier sequence or a molecular identifier sequence. An adapter can be added to the 5' end, to the 3' end, or to both ends of a molecule of a nucleic acid. An adapter can also be added as an extension of a primer. As used in the present description, a nucleic acid sequenced with an adapter can be used in subsequent biochemical processes, purification and/or analysis of the sample labelled with the adapter itself. In some cases, an "Y" adapter can be used containing a single-strand region and a double-strand region, wherein the nucleotide bases are not complementary, as described, for example, in US8,420,319.

By "Unique Molecular Identifier or UMI", we mean a sequence of nucleotides that depends on a target nucleic acid, where the sequence identifies the source of the amplified target nucleic acid, or a specific sample, as, for instance, a patient, whom the acid so-called target nucleic acid is derived from. During its use, every sample is labelled with a different UMI, and the labelled samples are processed and amplified. After sequencing the joined sample, UMI can be used to identify the source of the sequence. A UMI can be added to the 5' end of a polynucleotide or to the 3' end of a polynucleotide.

The term "reading" relates to the data output from a sequencer. A read sequence typically contains a string of nucleotide bases G (guanin), A (adenin), T (timin), and C (citosin), 50-1000 or more bases long, and every base of a read sequencer can be associated with a score indicating the quality of the "call" of said base. By "call", we mean assigning one of said nucleotide bases to a specific position in a sequence.

The term "reading group" relates to a group of sequences that have the same fragmentation interruption points and substantially identical sequences.

The term "consensus sequence" relates to a sequence that is obtained by aligning a set of readings in one region or in specific genome regions,and is defined as the most probable sequence that represents the base predominant in every position in the readings.

In the context of nucleic acid sequences, the terms "identical sequences" relates to the bases of two sequences that are the same when aligned for maximum correspondence.

As used in the present description before a list of components, characteristics, or elements, the terms "comprising", "comprises", "comprise" do not exclude or preclude the presence of other components, characteristics, elements in accordance with the techniques known to those skilled in the art.

In a first aspect, la present invention relates to a method for correcting systematic errors generated with any of the wet methods used for preparing immune repertoire libraries of immunoglobulins, starting from an extraction of genetic material, organized according to a workflow comprising the following steps:
a) generating a plurality of samples processed in parallel by way of a polymerase chain reaction (PCR), said libraries using at least one wet method;
b) purifying and sequencing the products of the polymerase chain reaction for generating sequences of nucleotides;
c) encoding the results of said step b) into two compressed text format files, each corresponding to the two sequencing directions, the forward one (5' - 3') and the reverse one (3' - 5') of every individual amplicon;
d) collecting said text format into consensus sequences;
e) assigning a pathological clone to the sequences thus obtained on the basis of a comparison with reference data banks;
f) correcting said systematic sequencing errors of the libraries, said step f) comprising the following sub-steps:
   g) aligning said text files with the sequencing of reference data banks, for a significant search for homology, account being taken of interruptions/deletions;
   h) generating counting matrices with the frequencies of the individual nucleotides observed depending on their position, in order to identify the mutations present in the immunoglobulins and their frequencies;
   i) eliminating those text files whose frequencies, as set in sub-step h), are lower than a predetermined threshold value, in order to obtain residual sequences;
   j) calculating the "Phred" quality score dependent on every position of said frequencies;
   k) correcting the positions of the nucleotides specific to immunoglobulins having said score lower than a predetermined threshold value, in order to obtain said corrected text files;
   l) differentiating said files according to the plurality of samples which they come from, processed in parallel as per step a);
   m) calculating the inverse Simpson index of said residual sequences.

In some embodiments, the start genetic material can be a DNA, a complementary DNA and/or an RNA. Methods for extracting a genetic material, for example from clinical samples, are well known to those skilled in the art. Samples comprise, for exemplary but not limitative purposes, body fluids, such as, for example, whole blood, fractioned blood, plasma, serum.

In particular embodiments, the molecules of nucleic acids of the sample can be 100 bp to 20 kb in length, even though fragments of at least 1 kb in length, for example 1 kb to 15 kb or 2 kb to 10 kb, can be used in many cases. The initial polymerase chain reaction can lead to multiples (for example trillions or more) of different amplicons. The concentration of the product obtained in this amplification step can be in a range from 0.05 ng/µl to 1 ug/µl, 0.5 ng/µl to 100 ng/µl. If necessary, the volume of use of the product in the subsequent step can be adjusted in accordance with the desired concentration. The polymerase used in the initial amplification step can be any type of polymerase having a 5' - 3' activity and a 3' - 5' exonuclease, stable at the temperatures reached during the cycles of said amplification. Examples of polymerase in the present description comprise, for exemplary but non-limitative purposes, Taq polymerase and Tth polymerase.

In particular embodiments, the compressed text file through which the obtained sequencing readings are encoded are the FASTQ files. After grouping the sequences according to the originate samples, a single consensus sequence is determined, which represents the most probable base in correspondence with every position in the readings for each of the reading groups obtained. In some embodiments, the most probable base is derived by counting the number of readings in the reading group with a given base call in a given position. In other embodiments, the most probable base is derived by using base quality scores, such as, for example, the Phred quality score. The number of readings per group can greatly vary and can range from 2 to 100 or more. Generating consensus sequences results in reducing the effects of sequencing errors (for example, wrong base calls) and errors caused by the polymerases used by incorporating incorrect bases, by providing a first data cleaning before the systematic error correction performed by the method of the present invention. One person skilled in the art will appreciate that these computational steps are illustrated in more details, for exemplary but non-limitative purposes, in the following figures and examples.

Any alignment algorithm can be used for interrogating a consensus sequence with respect to the reference sequence of the data banks. In particular embodiments, the data banks used is the IMGT interrogated through the IgBLAST algorithm.

In some embodiments of the present invention, the total number of readings in the reading group, the number of bases that correspond to the consensus base call, quality scores, and other data can be used for building up a statistic in the group of patients affected by CLL under observation, such as, for example, through the inverse Simpson index, for diagnostic and/or prognostic purposes. In particular embodiments of the present invention, a minimum threshold value of 1.2 has been set for the inverse Simpson index, in order to take account of all subclonal variants present in the population under study, not only the predominant ones.

All computational steps here described can be implemented on a computer and their corresponding instructions configured as programs for the method of the present invention.

The "hardware" architecture of said computer is well known to those skilled in the art and can comprise hardware components including one or several central processing units (CPU), a random access memory (RAM), a read only memory (ROM), a data storage medium, which is either internal and/or external (for example, a hard disk). Said computer might also comprise one or more graphic cards for processing and outputting graphical information via displaying means. Said components can be properly interconnected by using appropriate technologies, such as, for example, an internal "bus", well known to those skilled in the art. Said computer might also comprise interfaces suitable for communicating with general-use external components like a monitor, keyboard, mouse, printer, network, etc. In some embodiments, said computer might be part of a network configured for processing the method of the present invention in parallel, thus increasing computational power and speed. In some embodiments, the program read by the storage medium might be written in a memory equipped with an expanded card inserted in the computer, or in an expanded unit connected to the computer, in order to perform a part of or all operations according to the instructions of the method of the present invention.

In other embodiments, the method might be executed by using a "cloud computing" system. In these embodiments, data files and programming can be exported to a "cloud computer" which runs the program and returns an output to the user.

The memory of said computer can be any device capable of storing information for its retrieval by a processor and can comprise magnetic or optical devices or solid-state memory devices. A storage unit might comprise more than one physical storage device of equal or different types (for example, a storage unit might have several storage devices, such as multiple drives, cards, or multiple solid-state storage devices or any combination thereof). By permanent memory we mean, with reference to media readable by an electronic processor, a memory that is not deleted whenever electrical power is disconnected from a processor. Examples of permanent memory include, amongst others, hard disks, CD-ROM, and DVD-ROM. A Random Access Memory (RAM) is an example of non-permanent memory. A file present in a permanent memory can be editable and rewritable.

The operation of the computer is mainly controlled by an operating system, which is run by the central processing unit. The operating system can be stored in a system memory. In some embodiments, the operating system includes a file system. In addition to an operating system, a possible implementation of the system memory includes a variety of programming files and data files for implementing the method of the present invention. In some cases, programming might include a program, where the program can be formed of various modules, and a user interface module which enables a user to manually select or change the inputs or the parameters used by the program.

In some embodiments, the instructions according to the method of the present invention can be encoded on a computer-readable medium in a "programming" form, where the term "computer readable medium", as used here, relates to any storage or transmission media that provide instructions and/or data to a computer for running and/or processing purposes. Examples of storage media comprise hard disk, optical disk, magnetic-optical disk, CD-ROM, DVD-ROM, BLU-RAY, state-solid disk, and network attached storage (NAS), irrespective of whether such devices are internal or external to the computer or not. A file containing information can be stored on a computer-readable medium, where "recording" means recording information in such a way that it can be accessible and repeatable at a later moment.

The method implemented via computer according to the present invention can be executed by using programs that can be written in one or more computer programming languages known to those skilled in the art. Such languages comprise, for example, Python, Java and C++.

In all embodiments of the method according to the present invention, data can be forwarded to a "remote position", whereby "remote position" we mean a position other than the position where the program is run. For example, said other position might be another place (for example, an office, a laboratory, etc.) in the same town, another place in a different town, another place in a different State. Data can be sent from a given place to another place by way of a physical transportation of the medium containing said data (if possible) or by using other communication means, including radio or infrared transmission channels, or a network connection to another computer or device via electronic mail transmission and information recorded on websites and the like.

### INDUSTRIAL APPLICABILITY

One skilled in the art will appreciate that the method of the present invention can be used in a variety of applications, where said applications comprise nucleic acid sample analysis wherein it is necessary to detect genome mutations of immunoglobulins. The systematic errors correction according to the method of the present invention can allow for an estimate of the intraclonal diversification (ID) better than those of other methods known in the art, thus leading to a more effective prediction of treatment.

### EXAMPLES OF PREFERRED EMBODIMENTS

The present invention is illustrated in more details in a non-limitative manner by the following examples. For comparison purposes, one person skilled in the art will note a greater laboriousness of the procedures related to the present standardized methods that use UMI codes, both chemically and bioinformatically wise.

### EXAMPLE 1: Amplification protocols

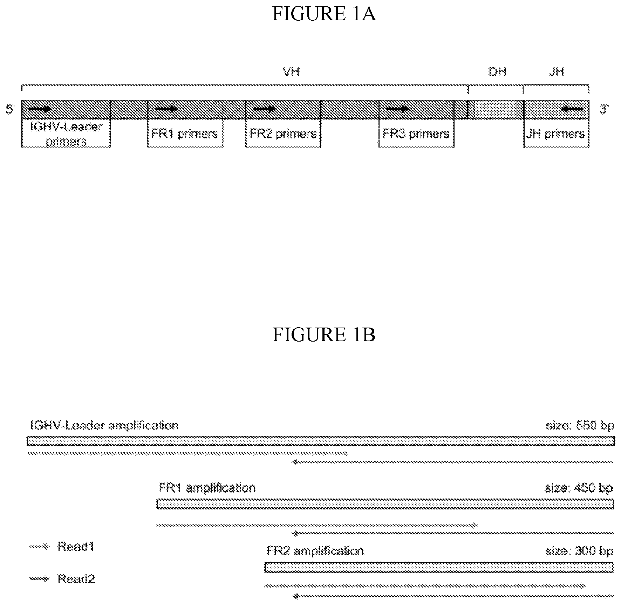

The IGHV sequencing of the method according to the present invention has been implemented by using a genome or complementary DNA as a start genetic material, with the help of specific primers for given V_{H} and J_{H} sequences, more specifically the LEADER regions, placed 150 bp above the start of the IGHV gene, and the FR1, FR2, FR3 consensus regions in combination with the J_{H} antisense primer, as substantially shown in Figure 1A, according to the Lymphotrack^{®} next generation method. Amplicons of different lengths have been obtained depending on the amplification protocol adopted, as substantially shown in Figure 1B. The libraries of the LEADER regions have been obtained from 923 patients by using 2 µL of a complementary DNA as a start genetic material, and the libraries of the FR1 regions from 168 patients by using 100 ng of a genome DNA as a start genetic material. The products obtained from the PCR reaction have been purified by using a PureLink Quick (ThermoFischer) kit to remove the primer in excess, non-incorporated nucleotide bases, salts, and enzymes, and diluted to a final concentration of 2 nM for a subsequent sequencing of the libraries. All samples have been sequenced by using the Illumina^{®} Miseq technology with flow cells by way of 2 cycles of 250 or 2 cycles of 300. Two compressed text files related to the two FASTQ reading sequence have been generated for every amplicon, more specifically one forward reading 1 (in the 5' - 3' direction of the strand) and an inverse reading 2 (in the 3' - 5' direction of the complementary strand), as substantially shown in Figure 1B. The FR2 region of IGHV resulted to be that, the one with two readings substantially most overlapable, and its corresponding protocol has consequently been used to perfect search for intraclonal diversification (ID) through the LEADER and FR1 regions of IGHV, as recommended by the European Research Initiative on CLL (ERIC) organization for patients affected by CLL.

### EXAMPLE 2: Analysis of the sequencing repertoire

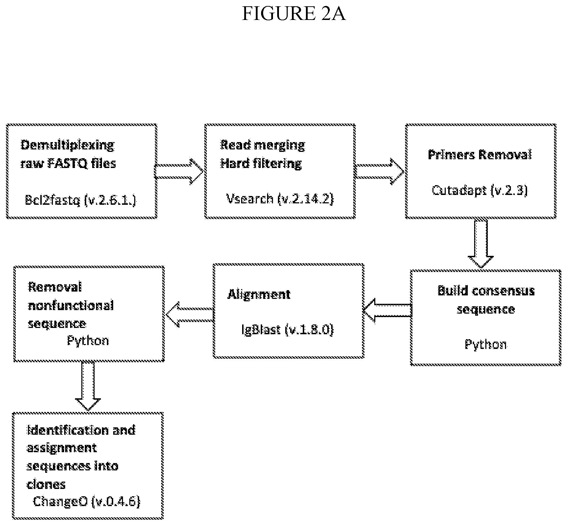

As substantially shown in Figure 2A, the FASTQ1 reading and the FASTQ2 reading of the billions of amplicons obtained from PCR have been differentiated ("demultiplexed") on a per originating sample basis and sequence similarity basis by using the bcl2fastq2 conversion software (v. 2.6.1) by Illumina^{®}. The vsearch (v. 2.14.2) program has been used to melt and clean from noise the two readings into one file by using the following commands: --fastq_mergepairs -fastq_minmergelen 5 -fastq_maxdiff 20; --fastq_filter - fastq_minlen 100 - fastq_maxee 3.0. The primers have been removed by using the cutadapt (v. 2.3) program by using the following commands (-p 0.15 -o 8 -discard-untrimmed) and the no-primer readings have been filtered away. The remaining reading groups, differentiated as a function of the originating samples, have been put together in one script by using the Python programming language in order to produce a consensus sequence. The consensus sequence has then been processed for alignment and search for homology by using the IgBLAST (v. 1.8.0) algorithm by comparison to nucleotide sequences contained in the IMGT data bank (updated as at August 17^{th} 2020). The sequences that have been found not to be functional to immunoglobulin have been discarded, and finally clone identification and assignment have been obtained by using the pRESTO (v. 0.6.2) and ChangeO (v. 0.4.6) programme package in order to identify the pathological clone of CLL. In particular, sequences featuring the same V_{H} and J_{H} regions, and having CDR3 regions of the same length, with a Hamming distance not less than 0.07, have been considered identical sequences belonging to one and the same clone.

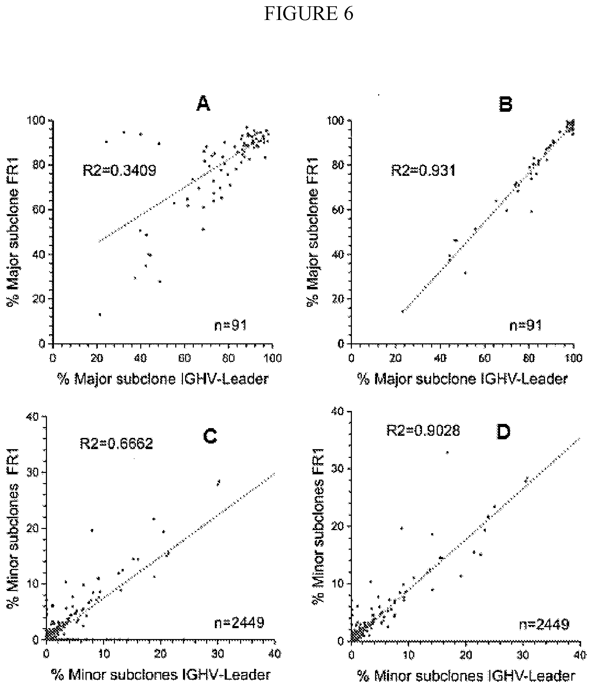

The mutations on the fatq sequences generated by the V_{H} LEADER and FR1 regions (with respect to the germline) have been identified, and the mutational frequency has been calculated as a percentage ratio of the number of mutated alleles observed to the number of total observations, both concerning the sequences most represented inside the pathological clone (major subclone) and the minority sequences (minor subclones), and determining a linear correlation coefficient between the two sets of primers, as substantially represented in Figures 6A and 6C.

### EXAMPLE 3: Systematic error correction in the sequencing repertoire

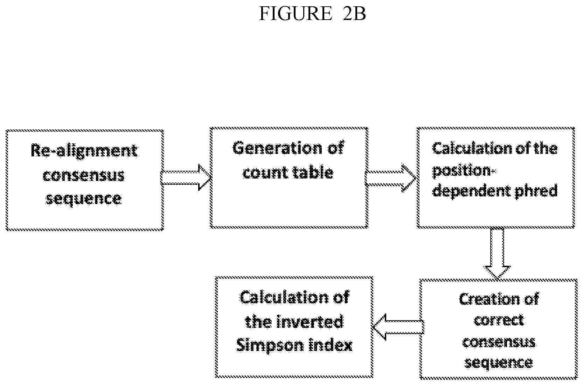

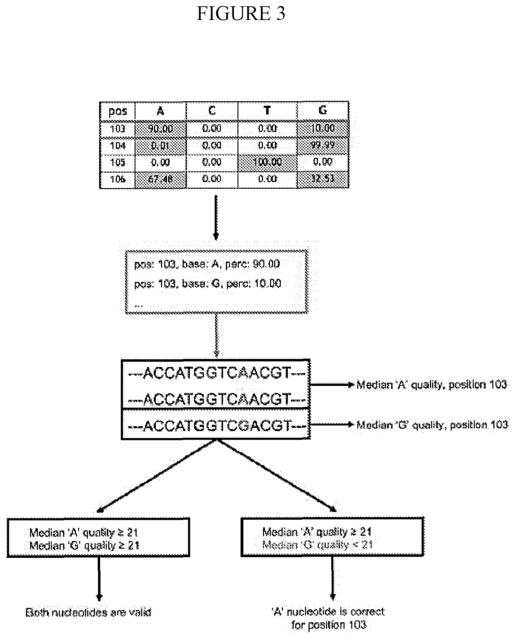

The subsequent bioinformatic operations have only been performed on those sequences which contain the pathological clone, aiming at eliminating the systematic errors generated during the sequencing process, and are subdivided into 5 packages as substantially shown in Figure 2B:
1) The consensus sequence obtained from the original fastq files has been interrogated for alignment and search for homology by using the IgBLAST algorithm, by comparison to nucleotide sequences contained in the IMGT data bank, this time also being taken account of interruptions and deletions.
2) An NxM matrix has been generated from the data of the previous step, where N = A, C, G, T, i.e. all possible nucleotides, and m = 1...n, all nucleotide positions of the immunoglobulins.
3) As substantially shown in Figure 3, only those positions where different nucleotides can be present with a frequency at least greater than 0.1% have been considered. Just for explanation, if 99.9% of the consensus sequences contains the G nucleotide base in a given position and the remaining 0.1% contains the nucleotide base A in the same position, then the two types of consensus sequences have been considered equally valid for systematic error correction. This in order to limit the weight of short chains in evaluating systematic errors. Then the "Phred" quality score has been calculated in correspondence with every position of the consensus sequences for assigning a specific nitrogenous base. A Phred score of 21 has been considered as a minimum threshold for an acceptable accuracy level. If the Phred score is lower than 21 in a given position, then its corresponding nucleotide has been considered a systematic base call error, and consequently corrected. The wrong nucleotide has been replaced by the second nucleotide more expressed in that position having the highest Phred score.
4) The remaining readings, differentiated on the basis of the originating samples and corrected for sequencing errors, have been collected in one script by using the Python programming language, in order to produce correct consensus sequences.
5) Finally, the inverse Simpson index (iSI) of the population of patients under study has been calculated starting from the correct consensus sequences for statistical and prognostic purposes. Only those clones which feature a total number of readings greater than 5000 have been considered in order to prevent an overestimate of said index.

The mutations on the fastq sequences (with respect to the germline) generated by the V_{H} LEADER and FR1 regions have been identified, and the mutational frequency has been calculated as a percentage ratio of the number of mutated alleles observed to the number of total observations, both concerning the most represented sequences internally to the pathological clone (major subclone) and the minority sequences (minor subclones), account being taken of the systematic error correction according to the protocol of the present invention and determining a linear correlation coefficient between the two sets of primers, as substantially shown in Figures 6B and 6D.

### REFERENCE EXAMPLE 1: Amplification protocols using molecular bar codes (UMI).

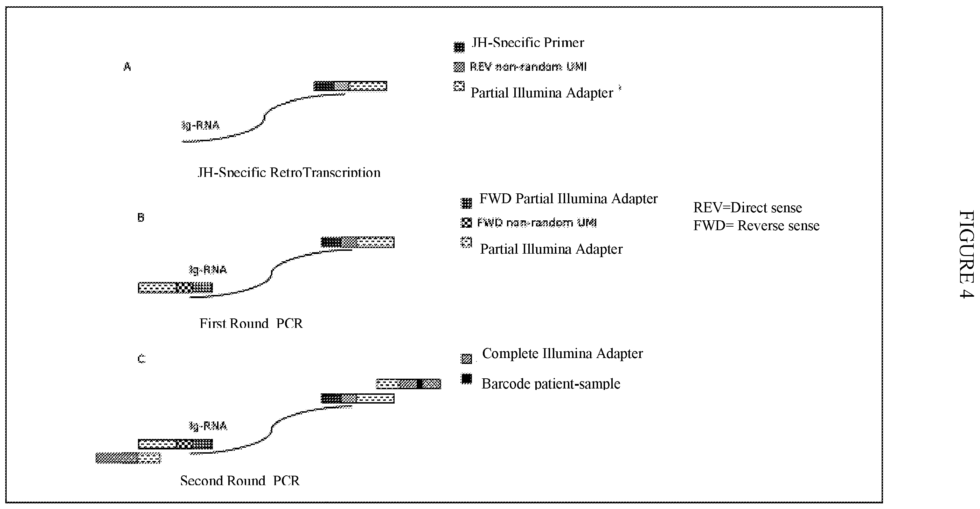

In order to compare the results obtained with the method of the present invention to those obtained with the present standardized methods that make use of molecular bar codes, 500 ng of RNA have been amplified by using specific UMI code-labelled J_{H} antisense primers and an adapter nucleotide sequence for a reverse transcription of the IGHV-related sequences only (as substantially shown in Figure 4A), through a single cycle at 25 °C for 5 minutes, 42°C for 60 minutes, and 72 °C for 15 minutes. Then the corresponding complementary DNA obtained has been amplified by using specific code-labelled V_{H} primers and Illumina^{®} partial adapters (GTTCTACAGTCCGACGATCG for the 5' - 3' transcription sense and TTGGCACCCGAGAATTCCAC for 3' - 5' sense) in order to prevent sequencing errors during the PCR reactions, as substantially shown in Figure 4B and in Table 1, by using a Verity (ThermoFischer) thermal cycler with a cycle at 98 °C for 30 seconds, 55 °C for two minutes, and 72 °C for 15 minutes). Finally, in order to prevent an excessive presence of short nucleotide sequences which might false the analysis made, 30000 molecule of the previous cycles have been further amplified by using specific indexed primers containing the P5 and P7 sequences of Illumina^{®} as substantially shown in Figure 4C, by using a Verity (ThermoFischer) thermal cycler, with the following protocol: 98 °C for one minute; 33 cycles at 98 °C of 20 seconds each, 33 cycles at 60 °C of 15 seconds each, 33 cycles at 72 °C of 15 minutes each. Every previously described amplification step requested a further purification stage by using the SPRI (Solid Phase Reversibile Immobilization) paramagnetic bed technology in order to remove reagents and primer in excess. All samples have been sequenced by using the Illumina^{®} Miseq technology with flow cells by means of 2 cycles of 300.

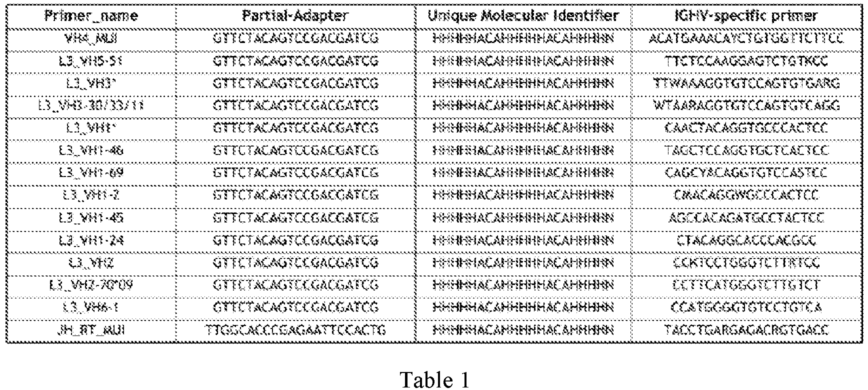

### REFERENCE EXAMPLE 2: Analysis of the sequencing repertoire with UMI

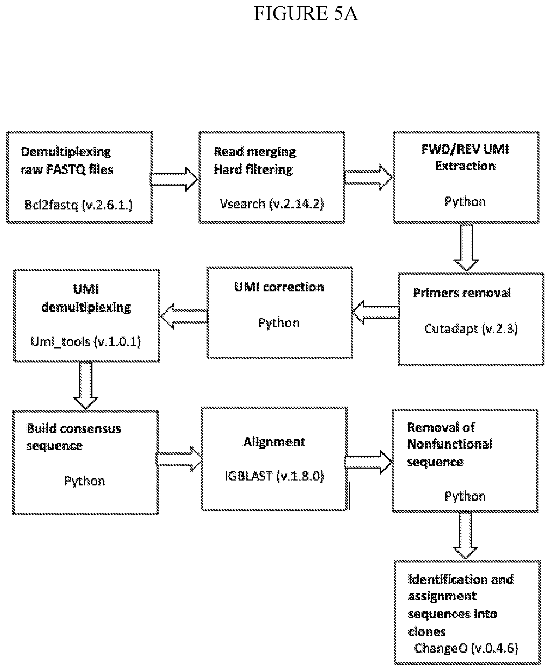

The fastq sequences obtained from the reference example 1 have been analysed with a bioinformatic approach of data readings, melting, and filtration, incomplete reading removal, alignment to data banks, and assignment of clonotypes depending on the originating samples substantially similar to that of example 1, as shown in Figure 5A.

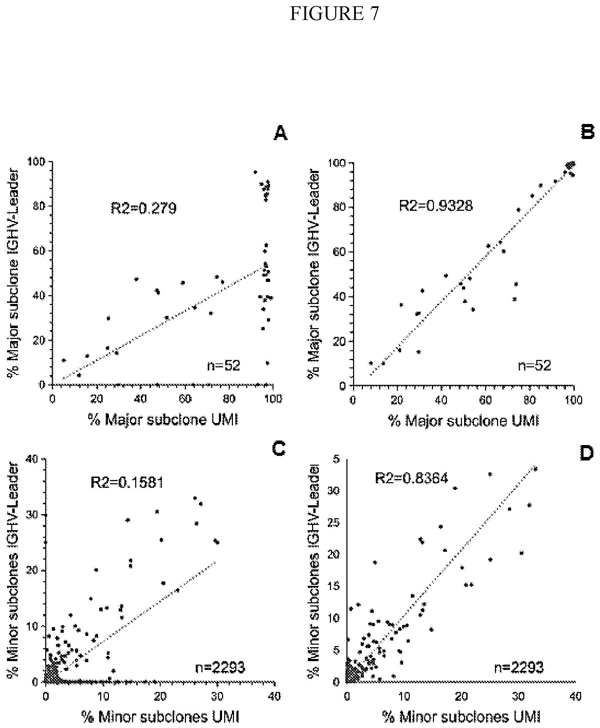

The mutations on the fastq sequences (with respect to the germline) generated by the V_{H} LEADER and FR1 regions through their corresponding UMI-labelled primers have been identified, and the mutational frequency has been calculated as a percentage ratio of the number of mutated alleles observed to the number of total observations, both concerning the sequences most represented internally to the pathological clone (major subclone) and the minority sequences (minor subclones), and determining a linear correlation coefficient between the two sets of primers, as substantially shown in Figures 7A and 7C.

### REFERENCE EXAMPLE 3: Systematic error correction of the UMI sequencing

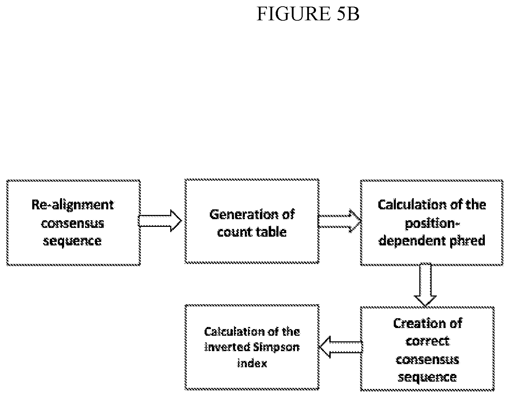

The systematic error correction on the data obtained from the reference Example 2 has been performed by using a bioinformatic approach substantially similar to that of Example 3, as shown in Figure 5B.

The mutations on the fastq sequences (with respect to the germline) generated by the LEADER and FR1 V_{H} regions through their corresponding UMI-labelled primers have been identified, and the mutational frequency has been calculated as a percentage ratio of the number of mutated alleles observed to the number of total observations, both concerning the sequences most represented internally to the pathological clone (major subclone) and the minority sequences (minor subclones), account being taken of the systematic error correction according to the protocol of the present invention and determining a linear correlation coefficient between the two sets of primers, as substantially shown in Figures 7B and 7D.

### EXAMPLE 4: Comparison of the mutational analyses.

An investigation has also been made on the mutational status of 759 patients affected by CLL with clinical data related to the Time to First Treatment (TTFT), by using in parallel the method of the present invention and a traditional sequencing method, for comparative purposes.

Out of these 759 cases, 396 had a mutational status "M" (i.e. >2% of mutations), whereas 363 had a so-called mutational status "UM" (<2% of mutations); according to the literature, the mutational status UM identified a sub-group of patients featuring a shorter TTFT, i.e. a worse prognosis, as compared to the cases having the "M" mutational status, as substantially represented in the following diagram A:

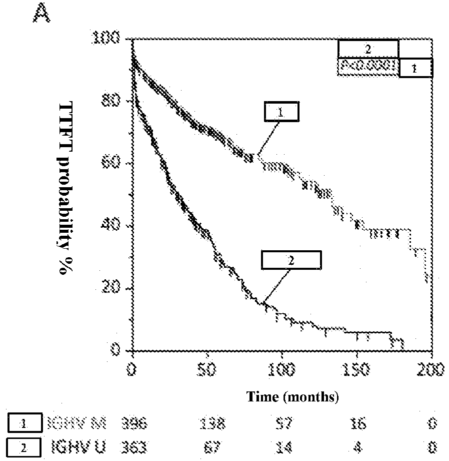

The method of the present invention made it possible to identify an optimal clinical parameter (cut-off) on the basis of statistical parameters known to those skilled in the art, corresponding to a value of the inverse Simpson index (iSI) of about 1.2, as shown in diagram B below; in particular, the identification of an optimal cut-off, TTFT, from a clinical point of view, has been made by applying the maximally selected log-rank statistics:

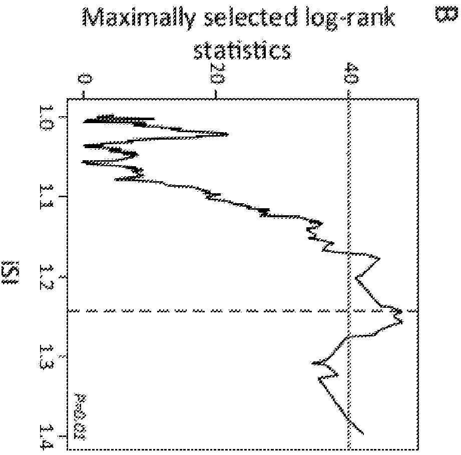

Furthermore, the application of the cut-off identified with the method of the present invention made it possible to split the group featuring the "M" mutational status of the patients affected by CLL into two sub-groups having different clinical courses: as substantially shown in the following diagram (C), the "M" cases featuring an iSI > 1.2 have a significantly better prognosis (n.1 in C) than the "M" cases featuring an iSI < 1.2 (n.2 in C); the following diagram also shows the "UM" cases, featuring worse clinical courses (n. 3 and n. 4 in C) for comparison purposes only.

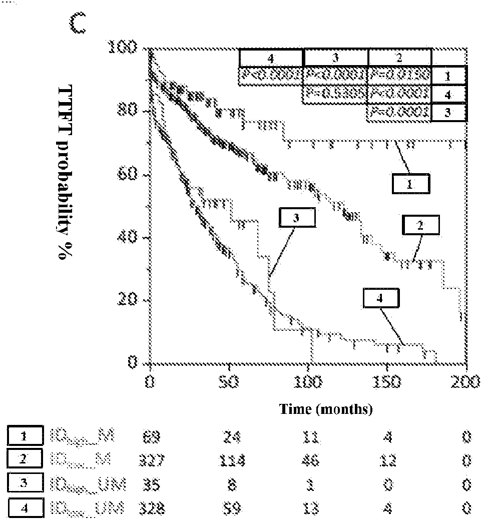

## Claims

1. A method for correcting systematic errors on data generated with any of the wet methods used for preparing immune repertoire libraries if immunoglobulins, starting from an extracted genetic material, organized according to a workflow comprising the following steps:
a) generating a plurality of samples processed in parallel by way of a polymerase chain reaction, said libraries using at least one wet method;
b) purifying and sequencing the products of the polymerase chain reaction for generating sequences of nucleotides;
c) encoding the results of said step b) into two compressed text format files, each corresponding to the two sequencing directions, the forward one (5' - 3') and the reverse one (3' - 5') of every individual amplicon, containing the pathological clone sequence;
d) collecting said text format into consensus sequences;
e) assigning a pathological clone to the sequences thus obtained on the basis of a comparison with reference data banks;
f) correcting said systematic sequencing errors of the libraries, said step f) comprising the following sub-steps:
g) aligning said text files with the sequencing of reference data banks, for a significant search for homology, account being taken of interruptions/deletions;
h) generating counting matrices with the frequencies of the individual nucleotides observed depending on their position, in order to identify the mutations present in the immunoglobulins and their frequencies;
i) eliminating those text files whose frequencies, as set in sub-step h), are lower than a predetermined threshold value, in order to obtain residual sequences;
j) calculating the "Phred" quality score dependent on every position of said frequencies;
k) correcting the positions of the nucleotides specific to immunoglobulins having said score lower than a predetermined threshold value, in order to obtain said corrected text files;
l) differentiating said files according to the plurality of samples which they come from, processed in parallel as per step a);
m) calculating the inverse Simpson index of said residual sequences;
**characterized in that** said method provides 1) information on a group of patients having the heavy chain variable region of immunoglobulin mutated, with an inverse Simpson index higher than a predetermined threshold value and 2) by way of such sequencing error correction implements a modification of the therapeutical plan of said group of patients, with an index of prognosis for intensifying the first treatment.

2. The method according to claim 1, wherein the systematic error correction step f) is performed on the consensus sequences of the pathological clone.

3. The method according to claims 1 and 2, wherein the correction step k) corrects all mutations having a Phred quality score less than 21.

4. The method according to claims 1 and 2, wherein the predetermined minimum threshold valued in step i) is 0.1%.

5. The method according to claims 1 and 2, wherein the inverse Simpson index is greater than 1.2.

6. The method according to claims 1 and 2, wherein said modification of the therapeutical plan comprises terminating the therapy.

7. The method according to any one of the previous claims for correcting library data related to heavy chains of immunoglobulins obtained through a next generation sequencing.

8. The method according to any one of the previous claims for a diagnosis and therapy of pathologies related to the immunological response of B lymphocytes.

## Patentansprüche

1. Verfahren zur Korrektur systematischer Fehler in Daten, die mit beliebigen Nassverfahren zur Herstellung von Immunrepertoirebibliotheken von Immunglobulinen erzeugt wurden, ausgehend von der Extraktion von genetischem Material, in einem Arbeitsablauf mit folgenden Schritten organisiert:
a) Erzeugung mehrerer Proben, die parallel mittels Polymerase-Kettenreaktion verarbeitet werden, wobei mindestens ein Nassverfahren von den Bibliotheken verwendet wird;
b) Reinigung und Sequenzierung der Produkte der Polymerase-Kettenreaktion zur Generierung von Nukleotidsequenzen;
c) Kodierung der Ergebnisse aus Schritt b) in zwei komprimierte Textdateien, die jeweils den beiden Sequenzierungsrichtungen, der Vorwärtsrichtung (5' - 3') und der Rückwärtsrichtung (3' - 5') jedes einzelnen Amplikons, entsprechen und die Sequenz des pathologischen Klons enthalten;
d) Zusammenführung der Textdateien zu Konsensussequenzen;
e) Zuordnung eines pathologischen Klons zu den so erhaltenen Sequenzen auf der Grundlage eines Vergleichs mit Referenzdatenbanken ;
f) Korrektur der systematischen Sequenzierungsfehler der Bibliotheken, wobei Schritt f) die folgenden Teilschritte umfasst:
g) Abgleich der Textdateien mit der Sequenzierung von Referenzdatenbanken zur Suche nach signifikanter Homologie unter Berücksichtigung von Unterbrechungen/Streichungen;
h) Erstellung von Zählmatrizen mit den Häufigkeiten der beobachteten einzelnen Nukleotide in Abhängigkeit von ihrer Position, um die in den Immunglobulinen vorhandenen Mutationen und ihre Häufigkeiten zu identifizieren;
i) Beseitigung derjenigen Textdateien, deren Häufigkeiten gemäß Teilschritt h) unter einem vorgegebenen Schwellenwert liegen, um Restsequenzen zu erhalten;
j) Berechnung des "Phred"-Qualitätswerts in Abhängigkeit von jeder Position der Häufigkeiten;
k) Korrektur der Positionen der für Immunglobuline spezifischen Nukleotide, deren Wert unter einem vorgegebenen Schwellenwert liegt, um die korrigierten Textdateien zu erhalten;
l) Unterscheidung der Dateien nach der Vielzahl der Proben, aus denen sie stammen, die gemäß Schritt a) parallel verarbeitet werden;
m) Berechnung des inversen Simpson-Index der Restsequenzen;
**dadurch gekennzeichnet, dass** das Verfahren 1) Informationen über eine Patientengruppe mit einer Mutation der variablen Region der schweren Kette des Immunglobulins liefert, deren inverser Simpson-Index über einem vorbestimmten Schwellenwert liegt, und 2) mittels dieser Sequenzierungsfehlerkorrektur eine Anpassung des Therapieplans dieser Patientengruppe mit einem Prognoseindex zur Intensivierung der Erstbehandlung ermöglicht.

2. Verfahren nach Anspruch 1, wobei der systematische Fehlerkorrekturschritt f) an den Konsensussequenzen des pathologischen Klons durchgeführt wird.

3. Verfahren nach Anspruch 1 und 2, wobei im Korrekturschritt k) alle Mutationen korrigiert werden, deren Phred-Qualitätswert kleiner als 21 ist.

4. Verfahren nach Anspruch 1 und 2, wobei der in Schritt i) vorbestimmte Mindestschwellenwert 0,1 % beträgt.

5. Verfahren nach Anspruch 1 und 2, wobei der inverse Simpson-Index größer als 1,2 ist.

6. Verfahren nach Anspruch 1 und 2, wobei die Änderung des Therapieplans die Beendigung der Therapie umfasst.

7. Verfahren nach einem der vorherigen Ansprüche zur Korrektur von Bibliotheksdaten in Bezug auf schwere Ketten von Immunglobulinen, die durch Sequenzierung der nächsten Generation gewonnen wurden.

8. Verfahren nach einem der vorhergehenden Ansprüche zur Diagnose und Therapie von Erkrankungen, die mit der Immunantwort von B-Lymphozyten zusammenhängen.

## Revendications

1. Procédé de correction des erreurs systématiques de donnés sur des données générées avec l'une quelconque des procédés humide utilisés pour la préparation de bibliothèques de répertoire immunitaire d'immunoglobulines, à partir d'une extraction de matériel génétique, organisé selon un flux de travail comprenant les étapes suivantes :
a) génération d'une pluralité d'échantillons traités en parallèle par réaction en chaîne par polymérase, lesdites bibliothèques utilisant au moins un procédé humide ;
b) purification et séquençage des produits de la réaction en chaîne par polymérase, pour générer des séquences de nucléotides ;
c) encodage des résultats de l'étape b) dans deux fichiers texte compressés, chacun correspondant aux deux sens de séquençage, le sens avant (5' - 3') et le sens arrière (3' - 5') de chaque amplicon, contenant la séquence du clone pathologique ;
d) assemblage de ces fichiers texte en séquences consensus ;
e) attribution d'un clone pathologique aux séquences ainsi obtenues sur la base d'une comparaison avec des banques de données de référence ;
f) correction desdites erreurs systématiques de séquençage des bibliothèques, ladite étape f) comprenant les sous-étapes suivantes :
g) alignement desdits fichiers texte avec le séquençage des banques de données de référence, pour une recherche significative d'homologie, en tenant compte des interruptions/suppressions !
h) génération de matrices de comptage avec les fréquences des nucléotides individuels observés en fonction de leur position, afin d'identifier les mutations présentes dans les immunoglobulines et leurs fréquences ;
i) élimination des fichiers texte dont les fréquences, telles que définies à la sous-étape h), sont inférieures à une valeur seuil prédéterminée, afin d'obtenir des séquences résiduelles ;
j) calcul du score de qualité « Phred » en fonction de chaque position de ces fréquences ;
k) correction des positions des nucléotides spécifiques aux immunoglobulines ayant ledit score inférieur à une valeur seuil prédéterminée, afin d'obtenir lesdits fichiers texte corrigés ;
l) différencier lesdits fichiers en fonction de la pluralité d'échantillons dont ils proviennent, traités en parallèle selon l'étape a) ;
m) calculer l'indice de Simpson inverse desdites séquences résiduelles ;
**caractérisé en ce que** ledit procédé fournit 1) des informations sur un groupe de patients présentant une mutation de la région variable de la chaîne lourde de l'immunoglobuline, avec un indice de Simpson inverse supérieur à une valeur seuil prédéterminée et 2) au moyen d'une telle correction d'erreur de séquençage mise en œuvre d'une modification du plan thérapeutique dudit groupe de patients, avec un indice de pronostic pour intensifier le premier traitement.

2. Procédé selon la revendication 1, dans lequel l'étape de correction d'erreur systématique f) est effectuée sur les séquences consensus du clone pathologique.

3. Procédé selon les revendications 1 et 2, dans lequel l'étape de correction k) corrige toutes les mutations ayant un score de mérite Phred inférieur à 21.

4. Procédé selon les revendications 1 et 2, dans lequel le seuil minimal prédéterminé évalué à l'étape i) est de 0,1 %.

5. Procédé selon les revendications 1 et 2, dans lequel l'indice de Simpson inverse est supérieur à 1,2.

6. Procédé selon les revendications 1 et 2, dans lequel ladite modification du plan thérapeutique consiste à mettre fin au traitement.

7. Procédé selon l'une quelconque des revendications précédentes pour la correction des données de bibliothèque relatives aux chaînes lourdes d'immunoglobulines obtenues par séquençage d'une prochaine génération.

8. Procédé selon l'une quelconque des revendications précédentes pour le diagnostic et le traitement des pathologies liées à la réponse immunologique des lymphocytes B.
